# EUROPÄISCHE PATENTSCHRIFT

(11) **EP 3 043 741 B1**
(45) Veröffentlichungstag und Bekanntmachung des Hinweises auf die Patenterteilung: **29.03.2017**
(21) Anmeldenummer: 13771079.4
(22) Anmeldetag: 13.09.2013
(51) Int. Cl.: A61B 1/00, A61B 1/04, A61B 1/24, A61C 13/08, A61C 13/00

(54) **VERFAHREN ZUR HERSTELLUNG EINES ZAHNERSATZES**
METHOD FOR PRODUCING A DENTAL PROSTHESIS
PROCÉDÉ DE FABRICATION D'UNE PROTHÈSE DENTAIRE

(43) Veröffentlichungstag der Anmeldung: 20.07.2016
(73) Patentinhaber: Heinzel, Ingo, 53121 Bonn (DE)
(72) Erfinder: Heinzel, Ingo, 53121 Bonn (DE)
(74) Vertreter: Wagner Albiger & Partner Patentanwälte mbB
(86) Internationale Anmeldenummer: PCT/EP2013/069017
(87) Internationale Veröffentlichungsnummer: WO 2015/036035

(56) Entgegenhaltungen:
- WO-A1-2009/105618
- WO-A1-2012/006717
- DE-A1- 10 203 664

## Beschreibung

### Stand der Technik

Die Erfindung betrifft ein Verfahren zur Herstellung eines Zahnersatzes. Solche Verfahren sind grundsätzlich bekannt.

Aus der Druckschrift DE 102 03 664 A1 ist zum Beispiel ein Verfahren zur Herstellung einer Zahnprothese unter Verwendung eines CAD/CAM Systems bekannt. Bei diesem Verfahren wird zum Zeitpunkt des gesunden Zustandes des Gebisses eine Fotoaufnahme gemacht, wobei die Zahnprothese später derart gestaltet wird, dass sie der Form zum Zeitpunkt des gesunden Zustandes gleicht.

Die erhaltenen Gestaltungsdaten der Zahnprothese werden zu einer Fräsbearbeitungsvorrichtung als Bearbeitungskommandos übertragen. Ein Blockmaterial wird einer Fräsbearbeitung unterzogen, um die Zahnprothese herzustellen. Bei diesem Verfahren ist es notwendig, eine Fotoaufnahme des Gebisses im gesunden Zustand zur Verfügung zu haben. Eine Herstellung der Zahnprothese ohne Fotografie des Gebisses im gesunden Zustand ist nachteiligerweise nicht möglich.

Aus der Druckschrift WO 2009/105618 A1 ist weiterhin ein Verfahren zur Herstellung eines Zahnersatz bekannt, bei dem zunächst eine digitale Fotografie der Zähne des Patienten aufgenommen wird. Danach wählt der Patient einen Zahnersatz aus. In einer Software werden in der Fotografie die existierenden Zähne des Patienten durch diejenigen Zähne ersetzt, die der Patient ausgewählt hat.

Der Patient kann danach den Zustand mit den neuen Zähnen betrachten. Bei Nichtgefallen wählt der Patient einen anderen Zahnersatz aus, der wiederum in der Software die alten Zähnen ersetzt. Diese Schritte des Auswählens und Vergleichens werden so lange durchgeführt, bis der Patient die gewünschte Zahnersatzform gefunden hat.

Nachdem die gewünschte Zahnersatzform gefunden wurde, werden die ermittelten Daten an den Hersteller des Zahnersatzes übergeben. Nachteiligerweise stehen dem Hersteller lediglich die Daten aus den Fotografien zur Verfügung, sodass die Umsetzung in eine dreidimensionales Modell für ein CAD/Cam System vergleichsweise ungenau ist.

Es ist daher die Aufgabe der vorliegenden Erfindung, ein Verfahren zur Herstellung eines Zahnersatzes bereitzustellen, das die Nachteile des Stands der Technik nicht aufweist und insbesondere eine optimale Anpassung des Zahnersatzes an die Wünsche des Patienten ermöglicht.

### Offenbarung der Erfindung

Die vorliegende Aufgabe wird mit einem Verfahren zur Herstellung eines Zahnersatzes und mit einem Zahnersatz gemäß den nebengeordneten Ansprüchen gelöst.

Das erfindungsgemäße Verfahren weist die folgenden Schritte auf:
-- Erstellen einer Abbildung des Gesichts des Patienten in einer Frontansicht, wobei der Mund des Patienten geöffnet ist und die zu ersetzende Zahnleiste des Patienten sichtbar ist,
-- Überblenden der Zähne des Patienten in der Abbildung des Gesichts des Patienten durch die digitale Fotografie einer ausgewählten Zahnleiste mittels einer Bildbearbeitungssoftware,
-- Anpassen der Größe und/oder der Form der ausgewählten Zahnleiste in der Fotografie an den Mund und/oder das Gesicht des Patienten mittels der Bildbearbeitungssoftware,
-- Übermittlung der durch die Anpassung erhaltenen Größe und/oder Form der ausgewählten Zahnleiste an ein CAD/CAM System,
-- Auswahl im CAD/CAM System einer dreidimensionalen Zahnleiste, die der ausgewählten Zahnleiste entspricht,
-- Anpassen der dreidimensionalen Zahnleiste im CAD/CAM System anhand der in der Fotografie ermittelten Größe und/oder Form der ausgewählten Zahnleiste,
-- Ausfräsen des Zahnersatzes aus einem Materialblock anhand der im CAD/CAM System ermittelten Größe und/oder Form der angepassten dreidimensionalen Zahnleiste.

Bei der Zahnleiste im Sinne der Erfindung kann es sich um lediglich einen einzigen Zahn handeln, es können aber auch zwei, drei oder mehr Zähne umfasst sein. Die Zahnleiste kann auch komplette Zahnreihen des Oberkiefers und/oder des Unterkiefers umfassen.

Mit Zahnersatz kann der endgültige Zahnersatz gemeint sein, der letztendlich in den Mund des Patienten eingesetzt wird. Zahnersatz im Sinne dieser Anmeldung kann aber auch lediglich ein Modell oder ein Provisorium (sog. Wax-up oder Mock-up Modelle) sein, welches z.B. zu Probezwecken in den Mund des Patienten eingesetzt und wieder entfernt werden kann.

Das erfindungsgemäße Verfahren hat u.a. den Vorteil, dass auch Provisorien erstellt werden können, sodass z.B. sofort der Sitz des Zahnersatzes ausprobiert werden kann, ohne dass gleich der endgültige Zahnersatz erstellt werden muss. Die letztendlich ermittelten Daten, wie Größe und/oder Form der angepassten dreidimensionalen Zahnleiste, können für verschiedene Modelle und Provisorien verwendet werden, ohne dass unbedingt eine neue Anpassung erfolgen muss. Dadurch werden Zeit und Kosten erheblich verringert.

Die Abbildung des Gesichts des Patienten kann grundsätzlich mit jedem bildgebenden Verfahren hergestellt werden. Die Abbildung kann z.B. eine digitale zweidimensionale Fotografie sein, ein Film, eine Zeichnung und/oder eine dreidimensionale Fotografie, welche z.B. mit einem Gesichtsscanner erstellt werden kann.

Das Überblenden der Zähne des Patienten durch die Fotografie der ausgewählten Zahnleiste erfolgt mittels einer geeigneten und aus dem Stand der Technik bekannten herkömmlichen Bildbearbeitungssoftware. Die ausgewählte Zahnleiste wird erfindungsgemäß derart an den Mund bzw. das Gesicht des Patienten angepasst, dass die ausgewählte Zahnleiste optimal zum Mund bzw. Gesicht des Patienten passt.

Dabei können medizinische Aspekte berücksichtigt werden. Außerdem können ästhetische Überlegungen angestellt werden, hinsichtlich derer die Form und/oder die Größe optimiert werden können. Der Patient kann unmittelbar erkennen, wie die ausgewählte Zahnleiste in seinem Mund aussehen würde, und kann seine Wünsche äußern.

So kann z.B. jeder einzelne Zahn in der Fotografie positioniert und bezüglich Form und/oder Größe verändert werden, bis er optimal in den Mund des Patienten bzw. in die Abbildung des Munds des Patienten passt. Dieses kann Zahn für Zahn durchgeführt werden, bis die Zahnleiste komplett angepasst ist.

Bei Nichtgefallen kann der Patient eine andere Zahnleiste auswählen. Dadurch ist vorteilhaft eine optimale Berücksichtigung der Patientenwünsche möglich. Auch hinsichtlich medizinischer und/oder ästhetischer Aspekte sind vorteilhaft optimale Anpassungen der Größe und/oder der Form der Zahnleiste möglich.

Die so erhaltene Größe und/oder Form der Zahnleiste bzw. die dazugehörigen Daten werden von der Bildbearbeitungssoftware erkannt und gegebenenfalls gespeichert. Diese Daten werden erfindungsgemäß an das CAD/CAM System übermittelt. Bei dem CAD/CAM System handelt es sich um eine aus dem Stand der Technik bekannte Software zur computergestützten Konstruktion und Herstellung eines Zahnersatzes. Der Zahntechniker kann mit einem solchen CAD/CAM System die Größe und/oder Form der Zähne bzw. der Zahnleiste verändern.

So kann z.B. in der Bildbearbeitungssoftware die Breite jedes einzelnen Zahns festgelegt und gemessen werden. Die gemessene Breit kann in das CAD/CAM System eingegeben werden, sodass auch im CAD/CAM System die optimal angepasste Breite verwendet werden kann. Diese Prozedur kann für jeden Zahn durchgeführt werden, bis die ganze Zahnleiste komplett angepasst ist.

Im CAD/CAM System ist in elektronischer Form das dreidimensionale Bild der ausgewählten Zahnleiste gespeichert bzw. enthalten. Dadurch ist es vorteilhaft möglich, die mit der Bildbearbeitungssoftware ermittelten Daten auf das dreidimensionale elektronische Modell im CAD/CAM System zu übertragen. Dadurch wird eine optimale Umsetzung der in der Bildbearbeitungssoftware ermittelten Größe und/oder Form in das CAD/CAM System erreicht.

Im CAD/CAM System ist die dreidimensionale Zahnleiste, die der fotografierten Zahnleiste entspricht, abgespeichert. Die Anpassung der Zahnleiste im CAD/CAM System kann automatisch erfolgen, sie kann aber auch manuell vom Zahntechniker vorgenommen werden. Nach endgültiger Festlegung bzw. nach endgültiger Bestimmung der Form und/oder Größe der dreidimensionalen Zahnleiste im CAD/CAM System können nun diese Daten an eine aus dem Stand der Technik bekannte Fräseinrichtung übergeben werden, die die Daten verarbeitet und den Zahnersatz ausfräst.

Der so hergestellte Zahnersatz entspricht nunmehr vorteilhaft den Wünschen des Patienten und erfüllt alle medizinischen und/oder ästhetischen Aspekte bzw. Anforderungen an den Zahnersatz.

Gemäß einer beispielhaften Ausführungsform der vorliegenden Erfindung ist vorgesehen, dass die Fotografie der Zahnleiste aus einer Zahnbibliothek ausgewählt wird, in der eine Vielzahl an Fotografien von unterschiedlichen Zahnleisten hinterlegt sind, wobei sich die Zahnleisten hinsichtlich ihrer Oberflächentexturen (z.B. Rillen auf den Zähnen), Zahnformen und/oder Zahngrößen unterscheiden.

Die Zahnbibliothek kann zum Beispiel ein gedruckter Katalog sein, aus dem sich der Patient nach seinen Wünschen die optimale Zahnleiste aussuchen kann. Die Zahnbibliothek kann auch in elektronischer Form vorliegen, so dass die unterschiedlichen Zahnleisten bzw. die unterschiedlichen Fotografien der Zahnleisten als Dateien bzw. Bilddateien vorliegen.

Dadurch ist es vorteilhaft möglich, dem Patienten eine große Auswahlmöglichkeit an Zahnleisten anzubieten, wodurch letztendlich die Patientenzufriedenheit vorteilhaft weiter verbessert werden kann. Auch medizinische Aspekte können bei der Auswahl der Zahnleiste aus der Zahnbibliothek natürlich berücksichtigt werden.

Gemäß einer weiteren beispielhaften Ausführungsform der vorliegenden Erfindung ist vorgesehen, dass die Fotografien der Zahnleisten in der Zahnbibliothek Fotografien von echten Zähnen sind. Echte Zähne heißt, dass die Fotografien von Zähnen von realen Personen bzw. Patienten gemacht worden sind. Dadurch wird vorteilhaft das Aussehen der Zahnleiste verbessert, weil die Zähne der Zahnleiste optimal zueinander passen.

Gemäß einer weiteren beispielhaften Ausführungsform der vorliegenden Erfindung ist vorgesehen, dass die Auswahl der Fotografie der Zahnleiste aus der Zahnbibliothek anhand von Gips- oder Kunststoffformen der fotografierten Zahnleisten erfolgt. Diese Gips- oder Kunststoffformen existieren zusätzlich zu den Fotografien der Zahnleisten.

Der Patient kann sich somit nicht nur die Fotografie anschauen, sondern er kann auch das gesamte dreidimensionale Aussehen der Zahnleiste in Augenschein nehmen. Dadurch ist vorteilhaft der Zahnersatz optimal an die Wünsche des Patienten anpassbar.

Gemäß einer weiteren beispielhaften Ausführungsform der vorliegenden Erfindung ist vorgesehen, dass die Gips- oder Kunststoffformen Formen von realen Zähnen sind, die identisch sind mit den entsprechenden fotografierten Zahnleisten in der Zahnbibliothek und den dreidimensionalen Zahnleisten im CAD/CAM System. Damit ist gemeint, dass die Gips- oder Kunststoffformen Formen von denselben realen Zähnen sind, die in den Fotografien der Zahnleisten dargestellt sind. Dadurch ist eine weitere Verbesserung der Herstellung des Zahnersatzes möglich.

Gemäß einer weiteren beispielhaften Ausführungsform der vorliegenden Erfindung ist vorgesehen, dass die Gips- oder Kunststoffformen in Formen von männlichen und weiblichen Personen unterteilt sind. Dadurch ist es vorteilhaft möglich, dass das unterschiedliche Aussehen von männlichen und weiblichen Zähnen bei der Auswahl der Zahnleiste und somit bei der Herstellung des Zahnersatzes berücksichtigt werden kann.

Gemäß einer weiteren beispielhaften Ausführungsform der vorliegenden Erfindung ist vorgesehen, dass das Gesicht des Patienten vermessen wird und in Abhängigkeit dieser Gesichtsvermessung die Größe und/oder die Form der ausgewählten Zahnleiste in der Fotografie bestimmt wird sowie die optimale Positionierung der Zähne erfolgen kann. Gesichtsvermessung heißt, dass verschiedene Punkte oder Linien bestimmt werden, die bei der Herstellung des Zahnersatzes berücksichtigt werden können. Dadurch ist eine verbesserte Anpassung der Zahnleiste an die Gegebenheiten des Mundes bzw. des Gesichts des Patienten sowie in ästhetischer und funktioneller Hinsicht möglich.

Gemäß einer weiteren beispielhaften Ausführungsform der vorliegenden Erfindung ist vorgesehen, dass die Gesichtsmitte des Patienten bestimmt wird und in Abhängigkeit der bestimmten Gesichtsmitte die Größe und/oder die Form der ausgewählten Zahnleiste in der Fotografie bestimmt wird. Die Gesichtsmitte im Sinne dieser Erfindung kann einen Punkt oder auch eine Linie umfassen.

Es handelt sich somit bei der Gesichtsmitte um eine Referenzlinie bzw. einen Referenzpunkt, die bzw. der zur Verbesserung des Zahnersatzes bzw. des Herstellungsverfahrens herangezogen werden kann. Die Gesichtsmitte muss nicht unbedingt den geometrischen Mittelpunkt bzw. die geometrische Symmetrielinie des Gesichts umfassen, es kann sich vielmehr auch um eine vom Zahntechniker ermittelte subjektive Referenzlinie bzw. einen ermittelten subjektiven Referenzpunkt handeln.

Gemäß einer weiteren beispielhaften Ausführungsform der vorliegenden Erfindung ist vorgesehen, dass zur Bestimmung der Gesichtsmitte die Pupillen des Patienten als Bezugspunkte genommen werden. Da die Pupillen des Patienten für die Symmetrie des Gesichts eine wichtige Rolle spielen, kann somit vorteilhaft das Aussehen der Zahnleiste erheblich verbessert werden.

Gemäß einer weiteren beispielhaften Ausführungsform der vorliegenden Erfindung ist vorgesehen, dass zur Bestimmung der Gesichtsmitte eine optische Vorrichtung verwendet wird. Eine solche Vorrichtung ist zum Beispiel aus der Druckschrift DE 20 2004 016 058 U1 bekannt, deren Offenbarung hiermit ausdrücklich in den Offenbarungsgehalt der Anmeldung aufgenommen wird. Mit einer solchen Vorrichtung kann zum Beispiel mit verbesserter Genauigkeit die senkrechte Gesichtsmitte des Patienten markiert werden.

Vorteilhaft ist somit eine weitere Verbesserung des Herstellungsverfahrens möglich.

Gemäß einer weiteren beispielhaften Ausführungsform der vorliegenden Erfindung ist vorgesehen, dass zur Bestimmung der Gesichtsmitte eine mit einer Rasterung versehene Fotografie des Gesichts verwendet wird. Damit ist gemeint, dass bei der Erstellung der Fotografie des Gesichts ein Raster z.B. aus vertikalen und horizontalen Linien vor das Gesicht gehalten wird, so dass auf der Fotografie Rasterlinien als Referenzlinien bzw. Rasterpunkte als Referenzpunkte verwendet werden können. Dadurch ist eine Verbesserung des Herstellungsverfahrens möglich.

Die (z.B. mit der aus der Druckschrift DE 20 2004 016 058 U1 bekannten Vorrichtung) ermittelte Gesichtsmitte kann auch in das CAD/CAM System eingegeben bzw. übertragen werden, sodass eine verbesserte Anpassung der Zahnleiste aufgrund der Möglichkeit, die Gesichtsmitte auch im CAD/CAM System als Referenzlinie zu verwenden, erreichbar ist.

Ein weiterer Gegenstand der vorliegenden Erfindung betrifft einen Zahnersatz, der mit einem Verfahren nach einem der vorhergehenden Ausführungsformen hergestellt worden ist. Ein solcher Zahnersatz wird aus den aus dem Stand der Technik bekannten herkömmlichen Materialien hergestellt. Zu den Vorteilen dieses Zahnersatzes wird auf die obigen Ausführungen zum Verfahren verwiesen.

Ein Ausführungsbeispiel der vorliegenden Erfindung ist in den Zeichnungen dargestellt und in der nachfolgenden Beschreibung näher erläutert.

Kurze Beschreibung der Zeichnungen
Figur 1 zeigt schematisch ein Blockdiagramm, das den Ablauf des erfindungsgemäßen Verfahrens gemäß einer beispielhaften Ausführungsform darstellt,
Figur 2, Figur 3, und Figur 4 zeigen jeweils eine Abbildungen eines Gesichts eines Patienten.

### Ausführungsformen der Erfindung

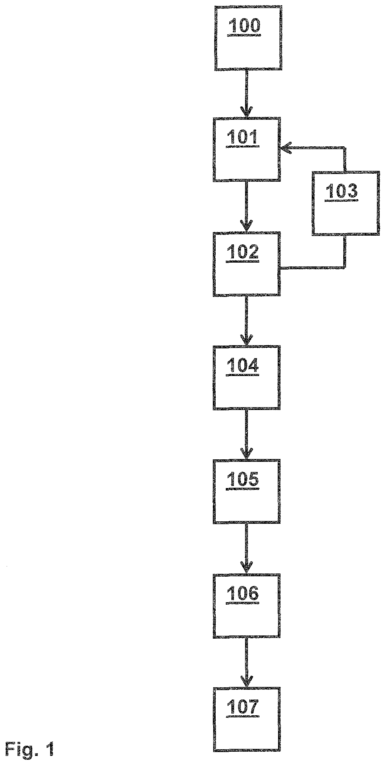

Figur 1 zeigt schematisch ein Blockdiagramm, das den Ablauf des erfindungsgemäßen Verfahrens gemäß einer beispielhaften Ausführungsform darstellt.

Der Block 100 symbolisiert das Erstellen einer digitalen Fotografie des Gesichts des Patienten in einer Frontansicht, wobei der Mund des Patienten geöffnet ist und die zu ersetzende Zahnleiste des Patienten sichtbar ist. Diese Fotografie zeigt sozusagen den aktuellen Zustand des Patienten mit den aktuellen Zähnen. Dabei können die aktuellen Zähne sichtbar sein, es können aber auch lediglich die bearbeiteten Zahnstümpfe zu sehen sein.

Es ist wichtig, dass der Mund des Patienten geöffnet ist und die aktuelle Zahnleiste sichtbar ist, damit im weiteren Verfahren die Ersatzzahnleiste in den geöffneten Mund eingesetzt werden kann. Die Fotografie des Gesichts kann auch bereits mit einer Rasterung versehen sein, die als Bezugspunkte bzw. Bezugslinien verwendet werden kann. Vorteilhaft wird in diesem Verfahrensschritt auch die Gesichtsmitte bestimmt. Dazu kann zum Beispiel die Position der Pupillen verwendet werden. Es kann aber auch eine herkömmliche Vorrichtung zur Bestimmung der Gesichtsmitte, wie aus der Druckschrift DE 20 2004 016 058 U1 bekannt, verwendet werden.

Der Block 101 symbolisiert die Auswahl einer Zahnleiste, die als Ersatz für die Zähne des Patienten verwendet werden soll. Der Patient kann zum Beispiel aus einer Zahnbibliothek, die in Form eines Katalogs vorliegt, die gewünschte Zahnleiste auswählen. Alternativ oder zusätzlich kann der Patient auch aus Gips- oder Kunststoffformen die gewünschte Zahnleiste auswählen.

Der Block 102 symbolisiert das Überblenden der Zähne des Patienten in der Fotografie des Gesichts des Patienten durch die digitale Fotografie der ausgewählten Zahnleiste mittels einer Bildbearbeitungssoftware. Dazu werden zum Beispiel die Fotografie des Gesichts und die Fotografie der ausgewählten Zahnleiste in die Bildbearbeitungssoftware eingefügt. Die Fotografie der ausgewählten Zahnleiste kann nun in der Bildbearbeitungssoftware über die aktuellen Zähne des Patienten gelegt werden.

Der Patient kann nun erkennen, wie die ausgewählte Zahnleiste in seinem Mund aussehen würde und kann entscheiden, ob ihm das Aussehen der Zahnleiste gefällt oder nicht. Bei Nichtgefallen geht das Verfahren zurück zum Block 101, so dass eine andere Zahnleiste zum Beispiel aus der Zahnbibliothek ausgewählt wird. Dieser Rückverweis ist mit dem Block 103 symbolisiert.

Wenn dem Patienten das Aussehen der Zahnleiste gefällt, so werden im Block 104 die Größe und/oder die Form der ausgewählten Zahnleiste in der der Bildbearbeitungssoftware an den Mund und/oder das Gesicht des Patienten angepasst. Bei dieser Anpassung ist es zum Beispiel hilfreich, wenn die Gesichtsmitte des Patienten bestimmt worden ist bzw. wenn das Gesicht des Patienten mit einem Raster versehen worden ist.

So können zum Beispiel einzelne Zähne von der Größe oder der Form her angepasst werden. Es können auch die Positionen der Zähne verändert werden. Bei dieser Anpassung der Zahnleiste können natürlich auch die Wünsche des Patienten berücksichtigt werden. Falls sich in diesem Stadium herausstellen sollte, dass die Zahnleiste dem Patienten doch nicht gefällt, so kann auch von diesem Verfahrensschritt aus auf den Block 101 zurückgegangen und eine neue Zahnleiste ausgewählt werden.

In diesem Verfahrensschritt bzw. Block 104 können weiterhin medizinische Aspekte berücksichtigt werden und der Zahntechniker kann weitere ästhetische Aspekte einbringen. Vorteilhaft ist es jederzeit möglich, das zukünftige Aussehen der Zahnleiste im Mund des Patienten betrachten zu können, so dass vorteilhaft eine optimale Anpassung der Zahnleiste möglich ist.

Nachdem der durch den Block 104 symbolisierte Verfahrensschritt abgeschlossen ist, werden die ermittelten Daten an ein herkömmliches CAD/CAM System übertragen. Diese Übertragung ist mit dem Block 105 symbolisiert und kann automatisch erfolgen, sie kann aber auch manuell durch den Zahntechniker ausgeführt werden. Nachdem die Daten an das CAD/CAM System übergeben worden sind, wird im CAD/CAM System derjenige dreidimensionale Datensatz aufgerufen, der der ausgewählten Zahnleiste entspricht.

Im CAD/CAM System ist für jede Zahnleiste, die ausgewählt werden kann, ein entsprechender Datensatz hinterlegt. Dieser Datensatz wird ausgewählt und in Abhängigkeit der von der Bildbearbeitungssoftware ermittelten Daten bearbeitet. Diese Bearbeitung wird mit dem Block 106 symbolisiert. Die gewünschte Größe und/oder Form wird somit auf das CAD/CAM System übertragen, so dass im CAD/CAM System die Darstellung der Zahnleiste bzw. die Größe und die Form der Zahnleiste mit der fotografischen Darstellung übereinstimmen.

Selbstverständlich kann der Zahntechniker auch in diesem Verfahrensschritt im CAD/CAM System die Größe und/oder die Form der Zahnleiste noch bearbeiten. Im letzten Verfahrensschritt werden dann die im CAD/CAM System endgültig ermittelten Bearbeitungsdaten an eine herkömmliche Fräseinrichtung übergeben, um letztendlich den gewünschten Zahnersatz herzustellen. Dieser Verfahrensschritt wird mit dem Block 107 symbolisiert. Das Herstellungsverfahren ist hiermit am Ende und der hergestellte Zahnersatz kann dem Zahnarzt zum Einsetzen in den Kiefer des Patienten übergeben werden.

Vorteilhaft beim erfindungsgemäßen Verfahren ist zudem, dass die Planung und die Gestaltung des Zahnersatzes, sowohl eines möglichen Wax-ups, Provisoriums und/oder des definitiven Zahnersatzes am Anfang des Verfahrens festgelegt wird, und dann über den gesamten Prozess hinweg, trotz erheblicher individueller/manueller Verfahrensschritte, beibehalten werden kann.

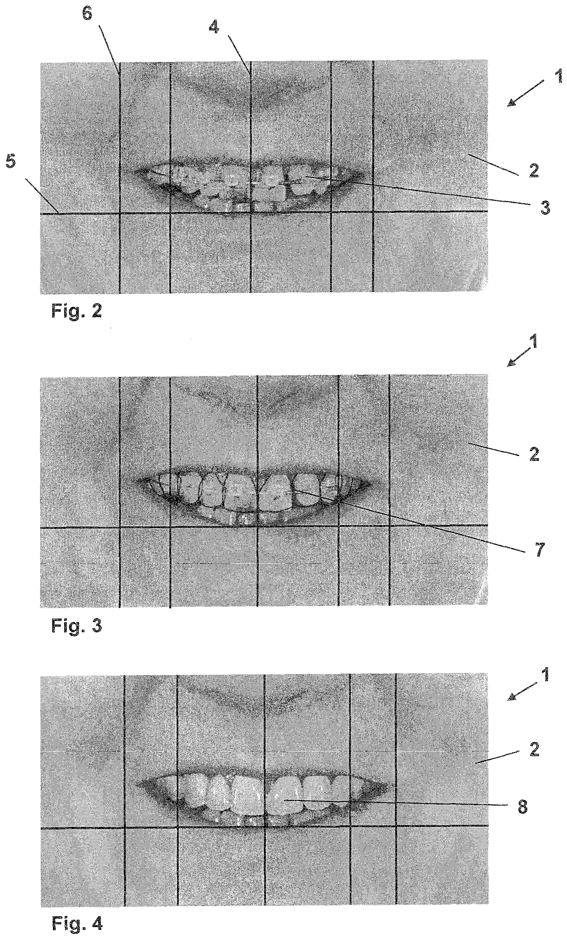

Figur 2 zeigt eine Abbildung 1 eines Gesichts 2 eines Patienten. Die Abbildung ist in diesem Falle eine digitale Fotografie. Die Figur 2 zeigt das Gesicht 2 des Patienten in einer Frontansicht, wobei sich die Zähne 3 des Patienten noch in der Situation mit den zu ersetzenden bzw. beschädigten Zähnen befinden. In diesem Falle sollen die Zähne 3 des Oberkiefers ersetzt werden. Die Zähne des Unterkiefers bleiben unverändert.

Zur Vermessung des Gesichts 2, des Mundes und der Zähne 3 werden Referenzlinien 4, 5, 6 in die Abbildung gezeichnet. In diesem Falle gibt es eine horizontale Referenzlinie 5 und fünf vertikale Referenzlinien 4, 6. Die horizontale Referenzlinie 5 verläuft in etwa durch den Punkt, an dem sich die tiefste Stelle der Oberkante der Unterlippe befindet.

Die ganz linke vertikale Referenzlinie verläuft durch die Pupille des Patienten. Die Pupillen sind in dieser Figur aus Gründen der besseren Darstellbarkeit nicht dargestellt. Die zweite vertikale Referenzlinie von links verläuft in etwa durch den zu ersetzenden Eckzahn des Patienten. Die mittlere horizontale bzw. vertikale Referenzlinie 4 entspricht in etwa der Gesichtsmitte 4 des Patienten. Die zweite vertikale Referenzlinie von rechts verläuft in etwa durch den weiteren zu ersetzenden Eckzahn des Patienten. Die ganz rechte vertikale Referenzlinie verläuft in etwa durch die weitere Pupille des Patienten. Die Figur 2 stellt den Ausgangszustand dar, in dem sich der Patient bzw. die Zähne des Patienten vor der Herstellung des Zahnersatzes befindet bzw. befinden.

Die Figur 3 zeigt wie die Figur 2 in einer Frontansicht das Gesicht 2 bzw. den Mund des Patienten in einer digitalen Fotografie. In der Fotografie befinden sich wie auch schon in der Figur 2 die dort beschriebenen Referenzlinien. In der Figur 3 sind Konturen 7 zu sehen, die den neuen Zähnen bzw. den Konturen 7 der ausgewählten Zahnleiste entsprechen. Die ausgewählte Zahnleiste bzw. die ausgewählten Zähne sind über die ursprünglichen Zähne geblendet.

Man kann in der Figur 3 erkennen, welche Unterschiede sich zwischen der ursprünglichen Situation und der Situation mit der ausgewählten Zahnleiste ergeben. Die Größe und die Form der Zähne wurden erfindungsgemäß derart angepasst, dass die gewünschten medizinischen und/oder ästhetischen Aspekte erfüllt sind und die Zähne in geeigneter Weise in den Mund des Patienten hineinpassen. Als weiterer Schritt könnten zum Beispiel nun die Konturen 7 der neuen Zähne vermessen werden. Diese Messergebnisse könnten dann in das CAD/CAM System eingegeben werden, damit die ermittelten Messwerte im dreidimensionalen Bild des CAD/CAM Systems umgesetzt werden können.

Figur 4 zeigt wie Figur 2 und Figur 3 das Gesicht 2 des Patienten in einer Frontansicht. In der Figur 4 sind die ausgewählten Zähne bzw. ist die ausgewählte Zahnleiste 8 in den Mund des Patienten eingesetzt, in dem die ursprünglichen Zähne 3 von der ausgewählten Zahnleiste 8 überblendet worden ist. An dieser Stelle des Verfahrens kann der Patient entscheiden, ob er mit der zukünftigen Situation der Zähne bzw. der Zahnleiste einverstanden ist. Falls nicht, kann eine andere Zahnleiste ausgewählt werden und das Verfahren beginnt von neuem.

## Patentansprüche

1. Verfahren zur Herstellung eines Zahnersatzes, aufweisend die folgenden Schritte:
-- Erstellen einer Abbildung (1) des Gesichts (2) des Patienten in einer Frontansicht, wobei der Mund des Patienten geöffnet ist und die zu ersetzende Zahnleiste (3) des Patienten sichtbar ist,
-- Überblenden der Zähne (3) des Patienten in der Abbildung (1) des Gesichts (2) des Patienten durch die digitale Fotografie einer ausgewählten Zahnleiste (8) mittels einer Bildbearbeitungssoftware,
-- Anpassen der Größe und/oder der Form der ausgewählten Zahnleiste (8) in der Fotografie an den Mund und/oder das Gesicht (2) des Patienten mittels der Bildbearbeitungssoftware,
-- Übermittlung der durch die Anpassung erhaltenen Größe und/oder Form der ausgewählten Zahnleiste (8) an ein CAD/CAM System,
-- Auswahl im CAD/CAM System einer dreidimensionalen Zahnleiste, die der ausgewählten Zahnleiste (8) entspricht,
-- Anpassen der dreidimensionalen Zahnleiste im CAD/CAM System anhand der in der Fotografie ermittelten Größe und/oder Form der ausgewählten Zahnleiste (8),
-- Ausfräsen des Zahnersatzes aus einem Materialblock anhand der im CAD/CAM System ermittelten Größe und/oder Form der angepassten dreidimensionalen Zahnleiste.

2. Verfahren nach Anspruch 1, **dadurch gekennzeichnet, dass** die Fotografie der Zahnleiste (8) aus einer Zahnbibliothek ausgewählt wird, in der eine Vielzahl an Fotografien von unterschiedlichen Zahnleisten hinterlegt sind, wobei sich die Zahnleisten hinsichtlich ihrer Zahnformen und/oder Zahngrößen unterscheiden.

3. Verfahren nach Anspruch 2, **dadurch gekennzeichnet, dass** die Fotografien der Zahnleisten (8) in der Zahnbibliothek Fotografien von echten Zähnen sind.

4. Verfahren nach einem der Ansprüche 2 oder 3, **dadurch gekennzeichnet, dass** die Auswahl der Fotografie der Zahnleiste (8) aus der Zahnbibliothek anhand von Gips- oder Kunststoffformen der fotografierten Zahnleisten erfolgt.

5. Verfahren nach Anspruch 4, **dadurch gekennzeichnet, dass** die Gips- oder Kunststoffformen Formen von realen Zähnen sind, die identisch sind mit den entsprechenden fotografierten Zahnleisten (8) in der Zahnbibliothek und den dreidimensionalen Zahnleisten im CAD/CAM System.

6. Verfahren nach Anspruch 5, **dadurch gekennzeichnet, dass** die Gips- oder Kunststoffformen in Formen von männlichen und weiblichen Personen unterteilt sind.

7. Verfahren nach einem der vorhergehenden Ansprüche, **dadurch gekennzeichnet, dass** das Gesicht (2) des Patienten vermessen wird und in Abhängigkeit dieser Gesichtsvermessung die Größe und/oder die Form der ausgewählten Zahnleiste (8) in der Fotografie bestimmt wird.

8. Verfahren nach Anspruch 7, **dadurch gekennzeichnet, dass** die Gesichtsmitte (4) des Patienten bestimmt wird und in Abhängigkeit der bestimmten Gesichtsmitte (4) die Größe und/oder die Form der ausgewählten Zahnleiste (8) in der Fotografie bestimmt wird.

9. Verfahren nach Anspruch 8, **dadurch gekennzeichnet, dass** zur Bestimmung der Gesichtsmitte (4) die Pupillen des Patienten als Bezugspunkte genommen werden.

10. Verfahren nach einem der Ansprüche 8 oder 9, **dadurch gekennzeichnet, dass** zur Bestimmung der Gesichtsmitte (4) eine optische Vorrichtung verwendet wird.

11. Verfahren nach einem der Ansprüche 8, 9 oder 10, **dadurch gekennzeichnet, dass** zur Bestimmung der Gesichtsmitte (4) eine mit einer Rasterung (5, 6) versehene Fotografie des Gesichts (2) verwendet wird.

## Claims

1. A method for producing a dental prosthesis, having the following steps:
-- Production of an image (1) of the front view of the patient's face (2) in which the patient's mouth is open and the set of the patient's teeth (3) being replaced is visible,
-- Superimposition of the patient's teeth (3) in the image (1) of the patient's face (2) by the digital photograph of a selected set of teeth (8) by means of image processing software,
-- Adaptation of the size and/or shape of the selected set of teeth (8) in the photograph to the patient's mouth and/or face (2) by means of the image processing software,
-- Transmission of the obtained size and/or shape of the selected set of teeth (8) to a CAD/CAM system,
-- Selection in the CAD/CAM system of a three-dimensional set of teeth that corresponds to the selected set of teeth (8),
-- Adaptation of the three-dimensional set of teeth in the CAD/CAM system based on the size and/or shape of the selected set of teeth (8) determined in the photograph,
-- Milling of the dental prosthesis out of a block of material based on the size and/or shape of the adapted three-dimensional set of teeth determined in the CAD/CAM system.

2. The method according to claim 1, **characterized in that** the photograph of the set of teeth (8) is selected from a tooth library in which a large number of photographs of different sets of teeth are stored, the sets of teeth differing with regard to their tooth shapes and/or tooth sizes.

3. The method according to claim 2, **characterized in that** the photographs of the sets of teeth (8) in the tooth library are photographs of real teeth.

4. The method according to one of claims 2 or 3, **characterized in that** the selection of the photograph of the set of teeth (8) from the tooth library is carried out based on plaster or plastic molds of the photographed sets of teeth.

5. The method according to claim 4, **characterized in that** the plaster or plastic molds are molds of real teeth that are identical to the corresponding photographed sets of teeth (8) in the tooth library and the three-dimensional sets of teeth in the CAD/CAM system.

6. The method according to claim 5, **characterized in that** the plaster or plastic molds are divided into molds of male and female individuals.

7. The method according to one of the preceding claims, **characterized in that** the patient's face (2) is measured and the size and/or shape of the selected set of teeth (8) in the photograph is/are established based on this facial measurement.

8. The method according to claim 7, **characterized in that** the center of the patient's face (4) is determined and the size and/or shape of the selected set of teeth (8) in the photograph is/are established based on the determined center of the face (4).

9. The method according to claim 8, **characterized in that** the patient's pupils are used as reference points for determining the center of the face (4).

10. The method according to one of claims 8 or 9, **characterized in that** an optical device is used to determine the center of the face (4).

11. The method according to one of claims 8, 9, or 10, **characterized in that** a photograph of the face (2) that is provided with a grid (5, 6) is used to determine the center of the face (4).

## Revendications

1. Procédé de fabrication d'une prothèse dentaire, comportant les étapes suivantes :
- élaboration d'une image (1) du visage (2) du patient en vue de face, la bouche du patient étant ouverte et la lame dentaire à remplacer (3) du patient étant visible,
- recouvrement des dents (3) du patient dans l'image (1) du visage (2) du patient par la photographie numérique d'une lame dentaire sélectionnée (8) au moyen d'un logiciel de traitement d'image,
- adaptation de la dimension et/ou de la forme de la lame dentaire sélectionnée (8) dans la photographie à la bouche et/ou au visage (2) du patient au moyen du logiciel de traitement d'image,
- transmission de la dimension et/ou de la forme, obtenues par l'adaptation, de la lame dentaire sélectionnée (8) à un système CAO/FAO,
- sélection, dans le système CAO/FAO, d'une lame dentaire tridimensionnelle qui correspond à la lame dentaire sélectionnée (8),
- adaptation de la lame dentaire tridimensionnelle dans le système CAO/FAO à l'aide de la dimension et/ou de la forme, déterminées dans la photographie, de la lame dentaire sélectionnée (8),
- fraisage de la prothèse dentaire à partir d'un bloc de matériau à l'aide de la dimension et/ou de la forme, déterminées dans le système CAO/FAO, de la lame dentaire tridimensionnelle adaptée.

2. Procédé selon la revendication 1, **caractérisé en ce qu'**on sélectionne la photographie de la lame dentaire (8) dans une bibliothèque dentaire dans laquelle sont mémorisées de nombreuses photographies de différentes lames dentaires, les lames dentaires se distinguant les unes des autres par leurs formes de dents et/ou par leurs dimensions de dents.

3. Procédé selon la revendication 2, **caractérisé en ce que** les photographies des lames dentaires (8) dans la bibliothèque dentaire sont des photographies de vraies dents.

4. Procédé selon l'une des revendications 2 ou 3, **caractérisé en ce que** la sélection de la photographie de la lame dentaire (8) dans la bibliothèque dentaire s'effectue à l'aide de moules en plâtre ou en plastique des lames dentaires photographiées.

5. Procédé selon la revendication 4, **caractérisé en ce que** les moules en plâtre ou en plastique sont des moules de vraies dents qui sont identiques aux lames dentaires (8) photographiées correspondantes dans la bibliothèque dentaire et aux lames dentaires tridimensionnelles dans le système CAO/FAO.

6. Procédé selon la revendication 5, **caractérisé en ce que** les moules en plâtre ou en plastique sont divisés en moules de personnes de sexe masculin et moules de personnes de sexe féminin.

7. Procédé selon l'une des revendications précédentes, **caractérisé en ce qu'**on mesure le visage (2) du patient et, en fonction de cette mesure de visage, on détermine la dimension et/ou la forme de la lame dentaire sélectionnée (8) dans la photographie.

8. Procédé selon la revendication 7, **caractérisé en ce qu'**on détermine le centre (4) du visage du patient et, en fonction du centre (4) déterminé du visage, on détermine la dimension et/ou la forme de la lame dentaire sélectionnée (8) dans la photographie.

9. Procédé selon la revendication 8, **caractérisé en ce que**, pour déterminer le centre (4) du visage, on prend comme points de référence les pupilles du patient.

10. Procédé selon l'une des revendications 8 ou 9, **caractérisé en ce que**, pour déterminer le centre (4) du visage, on utilise un dispositif otique.

11. Procédé selon l'une des revendications 8, 9 ou 10, **caractérisé en ce que**, pour déterminer le centre (4) du visage, on utilise une photographie du visage (2) qui est munie d'un quadrillage (5, 6).
